# FASCICULE DE BREVET EUROPEEN

(11) **EP 3 982 856 B1**
(45) Date de publication et mention de la délivrance du brevet: **12.03.2025**
(21) Numéro de dépôt: 20742808.7
(22) Date de dépôt: 15.06.2020
(51) Int. Cl.: A61B 17/70

(54) **DISPOSITIF DE FIXATION ADDITIONNEL D'UNE VIS PEDICULAIRE SUR UN SEGMENT OSSEUX RACHIDIEN**
VORRICHTUNG ZUR ZUSÄTZLICHEN BEFESTIGUNG EINER PEDIKELSCHRAUBE AN EINEM WIRBELSÄULENSEGMENT
DEVICE FOR ADDITIONALLY SECURING A PEDICLE SCREW TO A SPINAL BONE SEGMENT

(30) Priorité: 13.06.2019 FR 1906311
(43) Date de publication de la demande: 20.04.2022
(73) Titulaire: Medicrea International, 69140 Rillieux La Pape (FR)
(72) Inventeur: METAIZEAU, Jean-Damien Marie René, 21310 Arceau (FR); MOSNIER, Thomas, 69270 Rochetaillee Sur Saone (FR)
(74) Mandataire: Viering, Jentschura & Partner mbB Patent- und Rechtsanwälte
(86) Numéro de dépôt international: PCT/FR2020/051025
(87) Numéro de publication internationale: WO 2020/249918

(56) Documents cités:
- FR-A1- 2 992 847
- US-A- 6 086 590
- US-A1- 2003 163 133
- US-A1- 2016 249 957
- US-A1- 2017 265 906
- US-A1- 2018 353 217

## Description

La présente invention est relative à un dispositif de fixation additionnel permettant de relier au moins une vis pédiculaire d'un dispositif rachidien avec la vertèbre correspondante d'un segment rachidien afin de multiplier les points d'ancrage dudit dispositif rachidien sur une vertèbre.

On connait d'après le brevet antérieur FR3034979 un dispositif de fixation d'une vertèbre rachidienne sur une tige de liaison d'un dispositif rachidien comportant une vis pédiculaire et une attache de fixation solidaire d'une bague permettant la mise en place de la fixation d'une bande souple reliant la vertèbre.

Le dispositif de fixation complémentaire de l'art antérieur est réalisé au moyen d'une bande souple qui vient s'accrocher sur la tige d'ostéosynthèse du dispositif rachidien.

Ainsi, l'inconvénient du dispositif de fixation complémentaire de l'art antérieur consiste en ce qu'il permet d'assurer un ancrage additionnel qu'en fin d'opération, c'est-à-dire une fois le dispositif rachidien mis en place, l'addition de la fixation étant la dernière étape de la chirurgie, la tige étant déjà en place.

D'autres dispositifs de fixation complémentaire sont décrits dans US 2003/163133 A1, US 2018/353217 A1, et FR 2 992 847 A1.

Le dispositif de fixation additionnel vise à améliorer les points d'ancrage d'un dispositif rachidien avant la mise en place de la tige d'ostéosynthèse en assurant une liaison souple mise sous tension entre la vis pédiculaire et le corps de la vertèbre correspondante. L'invention est exposée dans les revendications annexées.

Le dispositif de fixation additionnel suivant la présente invention comprend un corps principal comprenant d'une part des premiers moyens de fixation coopérant directement ou indirectement avec le corps de la vis pédiculaire correspondante et d'autre part des seconds moyens de fixation assurant le passage et l'immobilisation d'un lien souple après mise sous tension de ce dernier afin de constituer un point d'ancrage additionnel.

Le dispositif de fixation additionnel suivant la présente invention comprend un corps principal qui est formé d'une douille dont les premiers moyens de fixation sont constitués d'un alésage central permettant son positionnement sur la tête intermédiaire à profil hémisphérique de la vis pédiculaire à double filetage, tandis que les seconds moyens de fixation sont disposés dans un prolongement latérale de ladite douille afin de positionner et immobiliser le lien souple dans un plan qui est décalé de l'axe vertical de ladite vis pédiculaire.

Le dispositif de fixation additionnel suivant la présente invention comprend un corps principal qui est formé d'un anneau ouvert présentant d'une part des premiers de fixation assurant l'immobilisation dudit anneau sur la périphérie externe de la tête hémisphérique de la vis pédiculaire à double filetage et d'autre part des seconds moyens de fixation disposés dans le prolongement latérale dudit anneau afin de positionner et immobiliser le lien souple dans un plan qui est décalé de l'axe vertical de ladite vis pédiculaire.

Le dispositif de fixation additionnel suivant la présente invention comprend des premiers moyens de fixation qui sont constitués d'une lame souple ouverte dont la face interne est pourvue d'une rainure coopérant avec des ergots aménagés sur la périphérie externe de la tête hémisphérique de la vis pédiculaire à double filetage, ladite lame souple ouverte comportant au niveau de sa première extrémité libre une ouverture, tandis que la seconde extrémité libre et opposée est solidaire des seconds moyens de fixation qui sont constitués d'un connecteur présentant à sa base une fente horizontale et suivant une direction verticale une vis de serrage.

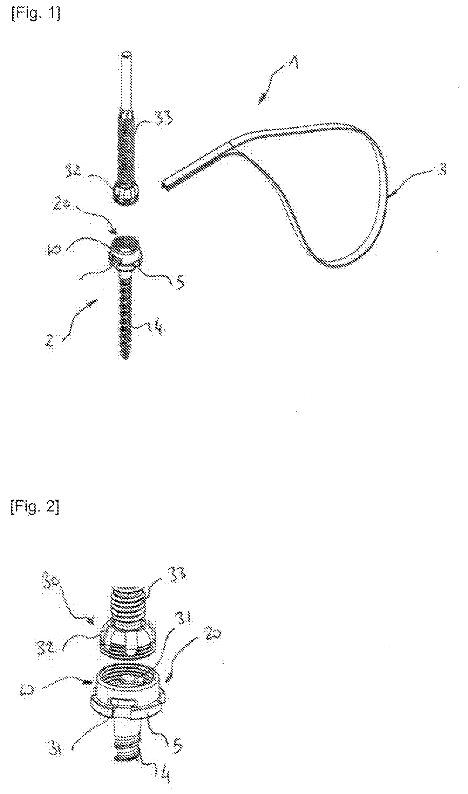

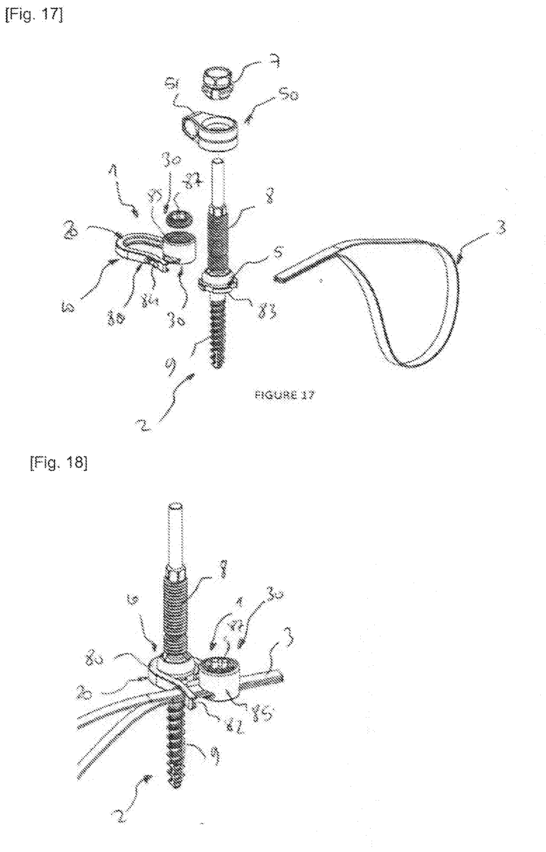

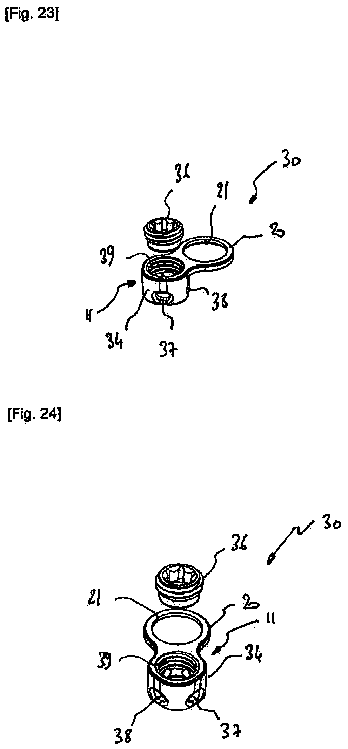

La description qui va suivre en regard des dessins annexés et donnés à titre d'exemples non limitatifs permettra de mieux comprendre l'invention, les caractéristiques qu'elle présente, et les avantages qu'elle est susceptible de procurer. Les figures 1 à 16 représentent des exemples qui aident à la compréhension de l'invention tandis que les figures 17 à 24 décrivent des réalisations de la présente invention :
[Fig. 1 à 3] sont des vues illustrant un premier mode de réalisation d'un exemple d'un dispositif de fixation additionnel agencé sur une vis pédiculaire à double filetage.
[Fig. 4 à 6] sont des vues montrant un second mode de réalisation de l'exemple du dispositif de fixation additionnel agencé sur la tête intermédiaire à profil hémisphérique d'une vis pédiculaire à double filetage.
[Fig. 7 à 8] sont des vues représentant une première variante du dispositif de fixation additionnel agencé sur la tête intermédiaire à profil hémisphérique d'une vis pédiculaire à double filetage suivant l'exemple.
[Fig. 9 à 14] sont des vues illustrant une seconde variante du dispositif de fixation additionnel agencé sur la périphérie externe de la tête hémisphérique d'une vis pédiculaire à double filetage suivant l'exemple.
[Fig. 15 et 16] sont des vues représentant une troisième variante du dispositif de fixation additionnel agencé sur la périphérie externe d'un connecteur de liaison d'un dispositif rachidien monté libre angulairement autour de la tête d'une vis pédiculaire suivant l'exemple.
[Fig. 17 à 20] sont des vues représentant une première variante du dispositif de fixation additionnel agencé sur la tête intermédiaire à profil hémisphérique d'une vis pédiculaire à double filetage suivant la présente invention.
[Fig. 21 à 24] sont des vues représentant une seconde variante du dispositif de fixation additionnel agencé sur la tête intermédiaire à profil hémisphérique d'une vis pédiculaire à double filetage suivant la présente invention.

On a montré en figures 1 à 3 un dispositif de fixation additionnel 1 permettant de relier au moins une vis pédiculaire 2 d'un dispositif rachidien 50 avec la vertèbre correspondante d'un segment rachidien non représenté afin de multiplier les points d'ancrage dudit dispositif rachidien sur une vertèbre.

Le dispositif de fixation additionnel 1 selon un premier mode de réalisation de l'exemple comprend un corps principal 10 comprenant d'une part des premiers moyens de fixation 20 qui se confondent avec le corps de la vis pédiculaire 2 et d'autre part des seconds moyens de fixation 30 assurant le passage et l'immobilisation d'un lien souple 3 après la mise sous tension de ce dernier afin de constituer un point d'ancrage additionnel au niveau de la vertèbre correspondante.

Les seconds moyens de fixation 30 du corps principal 10 sont positionnés coaxialement à l'axe principal de la vis pédiculaire 2. Les seconds moyens de fixation 30 du corps principal 10 sont constitués d'une part de fentes 31 traversant perpendiculairement le corps de la vis pédiculaire 2 permettant le passage du lien souple 3 et d'autre part d'un dôme fileté 32 venant se visser à l'intérieur dudit corps principal 1 pour le verrouillage dudit lien après sa mise sous tension.

Le dôme fileté 32 est solidaire d'un axe fileté 33 qui est disposé dans le prolongement de l'axe vertical de la vis pédiculaire 2 et dans une direction opposée à celle du filetage d'ancrage osseux 4 afin de constituer une vis pédiculaire à double filetage (figure 2).

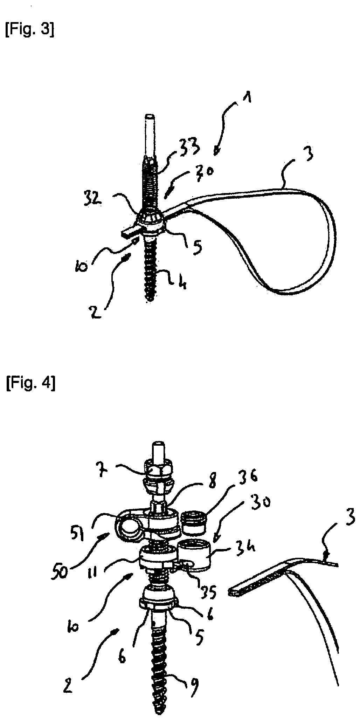

On a représenté en figures 4 à 6 le dispositif de fixation additionnel 1 selon un second mode de réalisation de l'exemple, agencé sur la tête intermédiaire à profil hémisphérique 5 d'une vis pédiculaire 2 à double filetage.

On entend par vis pédiculaire à double filetage 2, une vis comprenant de part et d'autre de la tête hémisphérique 5 et portée par le même axe principal un axe à filetage mécanique 8 et un axe d'ancrage osseux 9. L'axe à filetage mécanique 8 permet au moyen d'un écrou de serrage 7 d'immobiliser par exemple un connecteur de liaison 51 du dispositif rachidien 50.

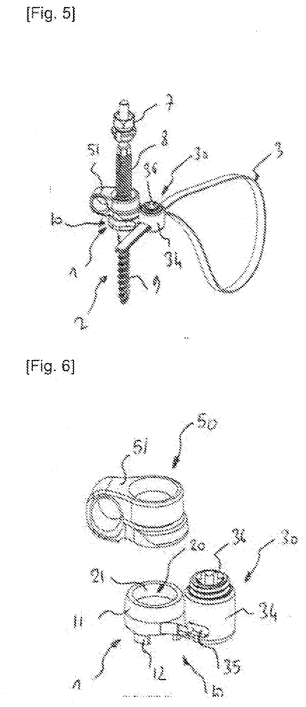

Le dispositif de fixation additionnel 1 comprend un corps principal 10 qui est formé d'une douille 11 comportant des premiers moyens de fixation 20 qui sont constitués d'un alésage central 21 permettant son positionnement sur la tête intermédiaire à profil hémisphérique 5 de la vis pédiculaire à double filetage 2 (figure 6).

On note que la douille 11 peut être indexée angulairement sur la tête hémisphérique 5 de la vis pédiculaire à double filetage 2. La douille peut comprendre des ergots 12 venant coopérer avec des encoches 6 ménagées sur la périphérie externe de la tête hémisphérique 5 (figure 6).

La douille 11 comporte des seconds moyens de fixation 30 qui sont disposés dans un prolongement latéral afin de positionner et immobiliser le lien souple 3 dans un plan qui est décalé de l'axe vertical de la vis pédiculaire 2.

Les seconds moyens de fixation 30 sont constitués dans le prolongement de la douille 11 d'un connecteur 34 présentant à sa base une fente horizontale 35 pour le passage du lien souple 3 et suivant une direction verticale une vis de serrage 36 permettant l'immobilisation dudit lien souple après sa mise en tension (figure 6).

La douille 11 du dispositif de fixation additionnel 1 est immobilisée en rotation et en translation verticale sur la tête hémisphérique 5 de la vis pédiculaire à double filetage 2 par l'intermédiaire de l'écrou de serrage 7 venant se visser sur l'axe de filetage mécanique 8 et sur lequel est préalablement agencé un connecteur de liaison 51.

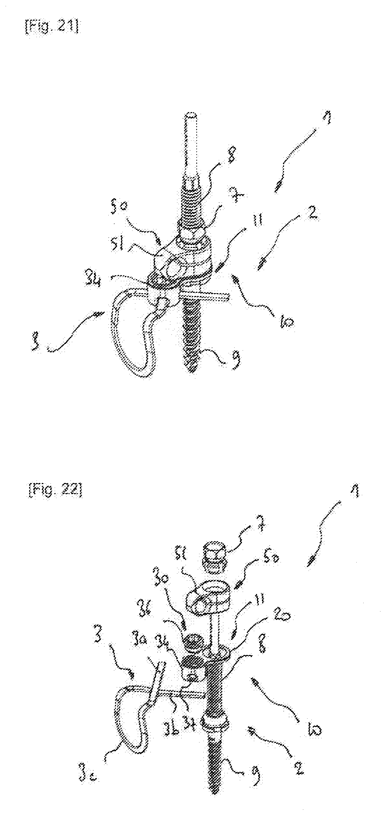

On a montré en figures 21 à 24 une variante de réalisation selon la présente invention du dispositif de fixation additionnel 1 et plus particulièrement de la douille 11 illustrée précédemment et agencée sur la tête intermédiaire à profil hémisphérique 5 de la vis pédiculaire à double filetage 2.

La douille 11 comporte des premiers moyens de fixation 20 qui sont constitués d'un alésage central 21 permettant son positionnement sur la tête intermédiaire à profil hémisphérique 5 de la vis pédiculaire à double filetage 2.

La douille 11 comporte des seconds moyens de fixation 30 qui sont disposés dans un prolongement latéral afin de positionner et immobiliser le lien souple 3 dans un plan qui est décalé de l'axe vertical de la vis pédiculaire 2.

Les seconds moyens de fixation 30 sont constitués dans le prolongement de la douille 11 d'un connecteur 34 présentant à sa base deux fentes horizontales 37 et 38 traversant de part en part ledit connecteur suivant deux directions axiales distinctes qui se coupent au centre dudit connecteur 34.

Les deux fentes horizontales 37, 38 permettent respectivement le passage des brins libres 3a, 3b du lien souple 3 afin que ces derniers se croisent à l'intérieur du connecteur 34 et ressortent dudit connecteur selon les directions axiales desdites fentes de manière que ledit lien souple 3 forme une boucle de retenue 3c.

Le connecteur 34 comporte au-dessus des fentes horizontales 37, 38 un alésage vertical fileté 39 destiné à coopérer avec une vis de serrage 36 permettant lors de son serrage l'immobilisation des brins 3a, 3b lien souple 3 à l'intérieur de la douille 11 après la mise en tension dudit lien souple.

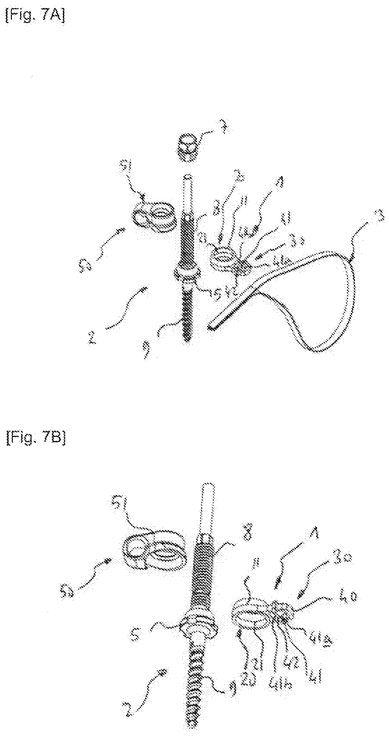

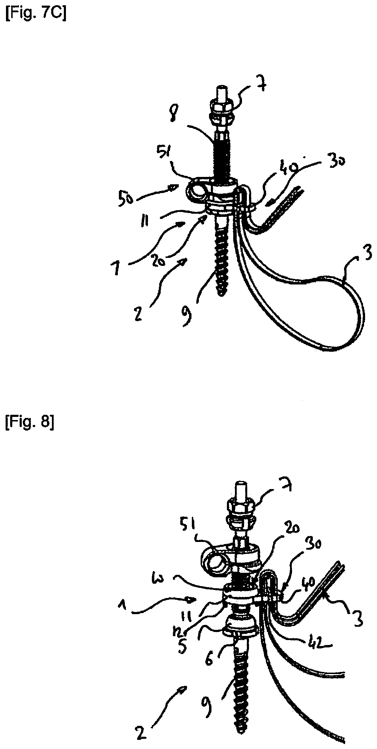

On a illustré en figures 7A à 7C et 8 une première variante du dispositif de fixation additionnel 1 selon l'exemple et plus particulièrement en ce qui concerne la douille 11 et son agencement sur la tête intermédiaire à profil hémisphérique 5 d'une vis pédiculaire à double filetage 2.

Les seconds moyens de fixation 30 de la douille 11 sont constitués d'un connecteur 40 présentant une ouverture centrale 41 positionnée dans un plan horizontal et séparée en deux parties distinctes par une bague de serrage 42 afin de délimiter deux passages verticaux 41a et 41b pour la mise en place et l'immobilisation du lien souple après sa mise en tension.

On a montré en 9 à 14 une seconde variante du dispositif de fixation additionnel 1 agencé sur la périphérie externe de la tête hémisphérique 5 d'une vis pédiculaire à double filetage 2 suivant l'exemple.

Le corps principal 10 du dispositif de fixation additionnel 1 est formé d'une collerette 60 présentant d'une part des premiers moyens de fixation 20 assurant l'immobilisation de ladite collerette 60 sur la périphérie externe de la tête hémisphérique 5 de la vis pédiculaire à double filetage 2 et d'autre part des seconds moyens de fixation 30 disposés dans le prolongement latéral de ladite collerette 60 afin de positionner et immobiliser le lien souple 3 dans un plan qui est décalé de l'axe vertical de ladite vis pédiculaire.

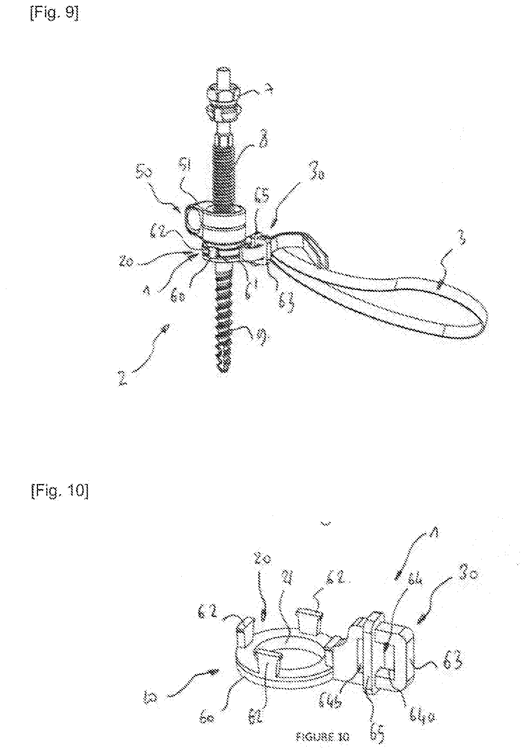

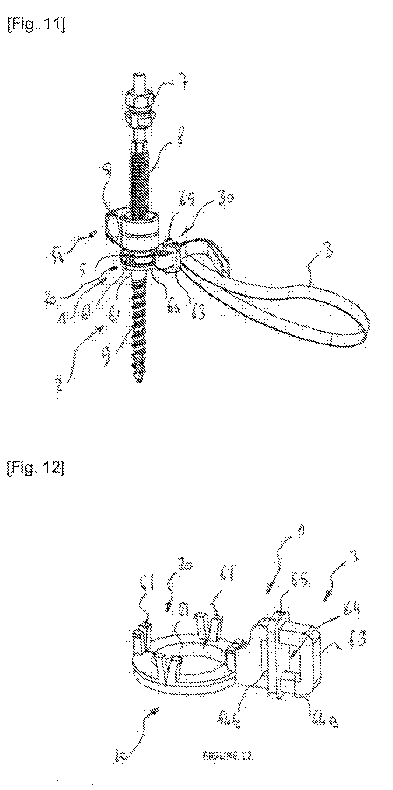

Les premiers moyens de fixation 20 de la collerette 60 peuvent être constitués d'éléments déformables 61 (figure 12) ou d'ergot 62 (figure 10) assurant l'immobilisation élastique sur la périphérie externe de la tête hémisphérique 5 de la vis pédiculaire à double filetage 2.

Les éléments déformables 61 ou ergots 62 coopèrent avec des encoches 6' aménagées sur la périphérie externe et inférieure de la tête hémisphérique 5.

En effet dans ce cas la collerette 60 est positionnée en traversant l'axe d'ancrage osseux 9 de la vis pédiculaire à double filetage 2 pour vernir se fixer sous la tête hémisphérique 5 (figures 9 à 12).

Les seconds moyens de fixation 30 de la collerette 60 sont constitués dans le prolongement de cette dernière d'un connecteur 63 présentant une ouverture centrale 64 positionnée dans un plan vertical et séparée en deux parties distinctes par une bague de serrage 65 afin de délimiter deux passages 64a et 64b pour la mise en place et l'immobilisation du lien souple 3 après sa mise en tension (figures 10 et 12).

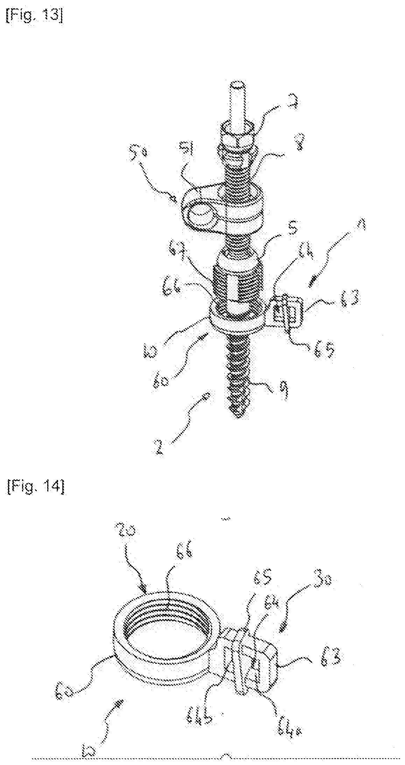

Selon une variante les premiers moyens de fixation 20 peuvent être constitués d'éléments mécaniques 67 de sorte que le collerette 60 comporte dans sa partie interne un filetage 66 assurant l'immobilisation par vissage sur la périphérie externe de la tête hémisphérique 5 de la vis pédiculaire à double filetage 2 (figures 13 et 14).

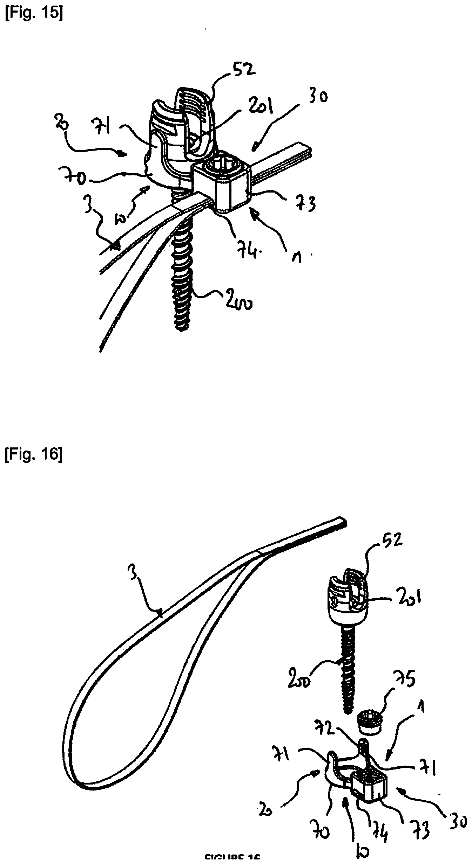

On a illustré en figures 15 et 16 une troisième variante selon l'exemple du dispositif de fixation additionnel 1 agencé sur la périphérie externe d'un connecteur de liaison 51 d'un dispositif rachidien 50 monté libre angulairement autour de la tête 201 d'une vis pédiculaire 200.

Le corps principal 10 du dispositif de fixation additionnel 1 est formé d'une bague 70 présentant des premiers moyens de fixation 20 assurant l'immobilisation de ladite bague sur la périphérie externe d'un connecteur de liaison en forme de U 52 monté libre angulairement autour de la tête 201 de la vis pédiculaire 200.

Les premiers moyens de fixation 20 sont constitués de branches verticales 71 déformables assurant l'immobilisation élastique sur la périphérie externe du connecteur de liaison 52 de la vis pédiculaire 200. Les branches verticales 71 sont solidaires au niveau de leur extrémité libre d'ergot 72 assurant la retenue de la bague 70 autour du connecteur de liaison 51 (figure 16).

La bague 70 comporte des seconds moyens de fixation 30 disposés dans le prolongement latéral de ladite bague afin de positionner et immobiliser le lien souple 3 dans un plan qui est décalé de l'axe vertical de ladite vis pédiculaire 200.

Les seconds moyens de fixation 30 sont constitués dans le prolongement de la bague 70 d'un connecteur 73 présentant à sa base une fente horizontale 74 pour le passage du lien souple 3 et suivant une direction verticale une vis de serrage 75 permettant l'immobilisation dudit lien souple après sa mise en tension (figure 16).

On a montré en figures 17 à 20 une première variante du dispositif de fixation additionnel 1 agencé sur la tête intermédiaire à profil hémisphérique 5 d'une vis pédiculaire à double filetage 2 suivant la présente invention.

Le corps principal 10 du dispositif de fixation additionnel 1 est formé d'un anneau ouvert 80 présentant des premiers moyens de fixation 20 assurant l'immobilisation dudit anneau ouvert 80 sur la périphérie externe de la tête hémisphérique 5 de la vis pédiculaire à double filetage 2.

L'anneau ouvert 80 comporte des seconds moyens de fixation 30 disposés dans le prolongement latéral afin de positionner et immobiliser le lien souple 3 dans un plan qui est décalé de l'axe vertical de ladite vis pédiculaire.

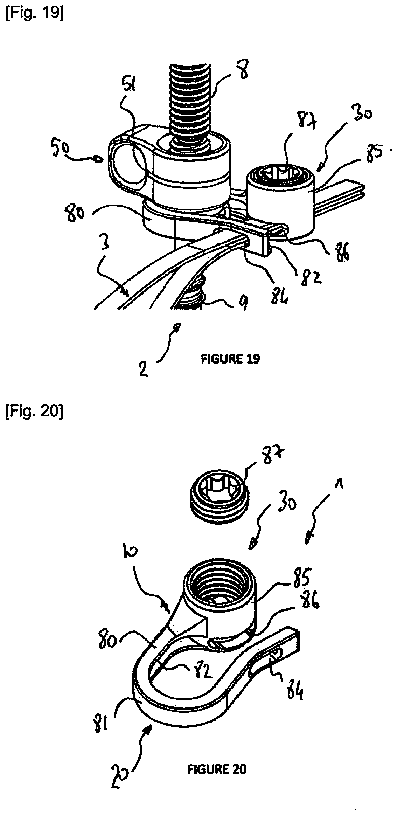

Les premiers moyens de fixation 20 sont constitués d'une lame souple ouverte et élastique 81 dont la face interne est pourvue d'une rainure 82 coopérant avec des ergots 83 aménagés sur la périphérie externe de la tête hémisphérique 5 de la vis pédiculaire à double filetage 2 (figure 20).

La lame souple ouverte 81 comporte au niveau de sa première extrémité libre une ouverture 84, tandis que la seconde extrémité libre et opposée est solidaire des seconds moyens de fixation 30 qui sont constitués d'un connecteur 85 présentant à sa base une fente horizontale 86 et suivant une direction verticale une vis de serrage 87 (figure 20).

Le lien souple 3 du dispositif de fixation additionnel 1 est introduit au travers de l'ouverture 84 et de la fente 86 permettant lors de sa mise sous tension le rapprochement de la lame souple 81 de l'anneau ouvert 80 autour de la tête hémisphérique 5, tandis que la vis de serrage 87 assure l'immobilisation du lien souple et donc le blocage de l'anneau ouvert autour de ladite tête hémisphérique (figure 19).

En effet dans ce cas la collerette 60 est positionnée en traversant l'axe d'ancrage osseux 9 de la vis pédiculaire à double filetage 2 pour vernir se fixer sous la tête hémisphérique 5 (figures 9 à 12).

Les seconds moyens de fixation 30 de la collerette 60 sont constitués dans le prolongement de cette dernière d'un connecteur 63 présentant une ouverture centrale 64 positionnée dans un plan vertical et séparée en deux parties distinctes par une bague de serrage 65 afin de délimiter deux passages 64a et 64b pour la mise en place et l'immobilisation du lien souple 3 après sa mise en tension (figures 10 et 12).

Selon une variante les premiers moyens de fixation 20 peuvent être constitués d'éléments mécaniques 67 de sorte que le collerette 60 comporte dans sa partie interne un filetage 66 assurant l'immobilisation par vissage sur la périphérie externe de la tête hémisphérique 5 de la vis pédiculaire à double filetage 2 (figures 13 et 14).

On a illustré en figures 15 et 16 une troisième variante du dispositif de fixation additionnel 1 agencé sur la périphérie externe d'un connecteur de liaison 51 d'un dispositif rachidien 50 monté libre angulairement autour de la tête 201 d'une vis pédiculaire 200.

Le corps principal 10 du dispositif de fixation additionnel 1 est formé d'une bague 70 présentant des premiers moyens de fixation 20 assurant l'immobilisation de ladite bague sur la périphérie externe d'un connecteur de liaison en forme de U 52 monté libre angulairement autour de la tête 201 de la vis pédiculaire 200.

Les premiers moyens de fixation 20 sont constitués de branches verticales 71 déformables assurant l'immobilisation élastique sur la périphérie externe du connecteur de liaison 52 de la vis pédiculaire 200. Les branches verticales 71 sont solidaires au niveau de leur extrémité libre d'ergot 72 assurant la retenue de la bague 70 autour du connecteur de liaison 51 (figure 16).

La bague 70 comporte des seconds moyens de fixation 30 disposés dans le prolongement latéral de ladite bague afin de positionner et immobiliser le lien souple 3 dans un plan qui est décalé de l'axe vertical de ladite vis pédiculaire 200.

Les seconds moyens de fixation 30 sont constitués dans le prolongement de la bague 70 d'un connecteur 73 présentant à sa base une fente horizontale 74 pour le passage du lien souple 3 et suivant une direction verticale une vis de serrage 75 permettant l'immobilisation dudit lien souple après sa mise en tension (figure 16).

On a montré en figures 17 à 20 une quatrième variante du dispositif de fixation additionnel 1 agencé sur la tête intermédiaire à profil hémisphérique 5 d'une vis pédiculaire à double filetage 2

Le corps principal 10 du dispositif de fixation additionnel 1 est formé d'un anneau ouvert 80 présentant des premiers moyens de fixation 20 assurant l'immobilisation dudit anneau ouvert 80 sur la périphérie externe de la tête hémisphérique 5 de la vis pédiculaire à double filetage 2.

L'anneau ouvert 80 comporte des seconds moyens de fixation 30 disposés dans le prolongement latéral afin de positionner et immobiliser le lien souple 3 dans un plan qui est décalé de l'axe vertical de ladite vis pédiculaire.

Les premiers moyens de fixation 20 sont constitués d'une lame souple ouverte et élastique 81 dont la face interne est pourvue d'une rainure 82 coopérant avec des ergots 83 aménagés sur la périphérie externe de la tête hémisphérique 5 de la vis pédiculaire à double filetage 2 (figure 20).

La lame souple ouverte 81 comporte au niveau de sa première extrémité libre une ouverture 84, tandis que la seconde extrémité libre et opposée est solidaire des seconds moyens de fixation 30 qui sont constitués d'un connecteur 85 présentant à sa base une fente horizontale 86 et suivant une direction verticale une vis de serrage 87 (figure 20).

Le lien souple 3 du dispositif de fixation additionnel 1 est introduit au travers de l'ouverture 84 et de la fente 86 permettant lors de sa mise sous tension le rapprochement de la lame souple 81 de l'anneau ouvert 80 autour de la tête hémisphérique 5, tandis que la vis de serrage 87 assure l'immobilisation du lien souple et donc le blocage de l'anneau ouvert autour de ladite tête hémisphérique (figure 19).

Il doit d'ailleurs être entendu que la description qui précède n'a été donnée qu'à titre d'exemple, et qu'elle ne limite nullement le domaine de l'invention dont on ne sortirait pas en remplaçant les détails d'exécution décrits par tout autre équivalent.

## Revendications

1. Dispositif de fixation additionnel (1), lien souple (3) et dispositif rachidien (50), dans lequel le dispositif de fixation additionnel (1) permet de relier au moins une vis pédiculaire à double filetage (2) du dispositif rachidien (50) avec le segment osseux correspondant avant la mise en place d'une tige d'ostéosynthèse dudit dispositif rachidien (50),
dans lequel le dispositif de fixation additionnel (1) comprend un corps principal (10) comprenant d'une part des premiers moyens de fixation (20) coopérant directement ou indirectement avec le corps de la vis pédiculaire correspondante (2) et d'autre part des seconds moyens de fixation (30) assurant le passage et l'immobilisation du lien souple (3) après mise sous tension de ce dernier afin de constituer un point d'ancrage additionnel,
dans lequel le corps principal (10) est formé d'une douille (11) dont les premiers moyens de fixation (20) sont constitués d'un alésage central (21) permettant son positionnement sur une tête intermédiaire à profil hémisphérique (5) de la vis pédiculaire à double filetage (2), tandis que les seconds moyens de fixation (30) sont disposés dans un prolongement latérale de ladite douille (11) afin de positionner et immobiliser le lien souple (3) dans un plan qui est décalé de l'axe vertical de ladite vis pédiculaire (2),
**caractérisé en ce que** les seconds moyens de fixation (30) sont constitués dans le prolongement de la douille (11) d'un connecteur (34) présentant à sa base deux fentes horizontales (37 ; 38) traversant de part en part ledit connecteur suivant deux directions axiales distinctes qui se coupent au centre dudit connecteur (34).

2. Dispositif de fixation additionnel (1) suivant la revendication 1,
**caractérisé en ce que** le connecteur (34) comporte au-dessus des fentes horizontales (37, 38) un alésage vertical fileté (39) destiné à coopérer avec une vis de serrage (36) permettant lors de son serrage l'immobilisation dudit lien souple (3) à l'intérieur de la douille (11) après la mise en tension dudit lien souple.

3. Dispositif de fixation additionnel (1), lien souple (3) et dispositif rachidien (50), dans lequel le dispositif de fixation additionnel (1) permet de relier au moins une vis pédiculaire à double filetage (2) du dispositif rachidien (50) avec le segment osseux correspondant avant la mise en place d'une tige d'ostéosynthèse dudit dispositif rachidien (50),
dans lequel le dispositif de fixation additionnel (1) comprend un corps principal (10) comprenant d'une part des premiers moyens de fixation (20) coopérant directement ou indirectement avec le corps de la vis pédiculaire correspondante (2) et d'autre part des seconds moyens de fixation (30) assurant le passage et l'immobilisation du lien souple (3) après mise sous tension de ce dernier afin de constituer un point d'ancrage additionnel,
dans lequel le corps principal (10) est formé d'un anneau ouvert (80) présentant d'une part les premiers de fixation (20) assurant l'immobilisation dudit anneau sur la périphérie externe d'une tête hémisphérique (5) de la vis pédiculaire à double filetage (2) et d'autre part des seconds moyens de fixation (30) disposés dans le prolongement latérale dudit anneau (80) afin de positionner et immobiliser le lien souple (3) dans un plan qui est décalé de l'axe vertical de ladite vis pédiculaire, **caractérisé en ce que** les premiers moyens de fixation (20) sont constitués d'une lame souple ouverte (81) dont la face interne est pourvue d'une rainure (82) coopérant avec des ergots (83) aménagés sur la périphérie externe de la tête hémisphérique (5) de la vis pédiculaire à double filetage (2), ladite lame souple ouverte (81) comporte au niveau de sa première extrémité libre une ouverture (84), tandis que la seconde extrémité libre et opposée est solidaire des seconds moyens de fixation (30) qui sont constitués d'un connecteur (85) présentant à sa base une fente horizontale (86) et suivant une direction verticale une vis de serrage (87).

## Patentansprüche

1. Zusätzliche Befestigungsvorrichtung (1), flexibles Verbindungsteil (3) und Vorrichtung (50) für eine Wirbelsäule, wobei die zusätzliche Befestigungsvorrichtung (1) es ermöglicht, mindestens eine Doppelgewindepedikelschraube (2) der Vorrichtung (50) für eine Wirbelsäule mit dem entsprechenden Knochensegment vor der Installation eines Osteosynthesestabs der Vorrichtung (50) für die Wirbelsäule zu verbinden,
wobei die zusätzliche Befestigungsvorrichtung (1) einen Hauptkörper (10) umfasst, umfassend einerseits erste Befestigungsmittel (20), die direkt oder indirekt mit dem Körper der entsprechenden Pedikelschraube (2) zusammenwirken, und andererseits zweite Befestigungsmittel (30), die den Durchgang und die Blockierung des flexiblen Verbindungsteils (3) nach einem Unterspannungsetzen des Letzteren gewährleisten, um einen zusätzlichen Verankerungspunkt zu bilden,
wobei der Hauptkörper (10) aus einer Hülse (11) ausgebildet ist, deren erste Befestigungsmittel (20) aus einer zentralen Bohrung (21) gebildet sind, die ihre Positionierung auf einem Zwischenkopf (5) mit halbkugelförmigem Profil der Doppelgewindepedikelschraube (2) ermöglichen, während die zweiten Befestigungsmittel (30) in einer seitlichen Verlängerung der Hülse (11) angeordnet sind, um das flexible Verbindungsteil (3) in einer Ebene zu positionieren und blockieren, die von der vertikalen Achse der Pedikelschraube (2) versetzt ist,
**dadurch gekennzeichnet, dass** die zweiten Befestigungsmittel (30) aus der Verlängerung der Hülse (11) einer Steckverbindung (34) gebildet sind, die an ihrer Basis zwei horizontale Schlitze (37; 38) vorweist, die in zwei unterschiedlichen axialen Richtungen, die sich in der Mitte der Steckverbindung kreuzen, durch die gesamte Steckverbindung (34) verlaufen.

2. Zusätzliche Befestigungsvorrichtung (1) nach Anspruch 1,
**dadurch gekennzeichnet, dass** die Steckverbindung (34) oberhalb der horizontalen Schlitze (37, 38) eine vertikale Gewindebohrung (39) aufweist, die dafür bestimmt ist, mit einer Spannschraube (36) zusammenzuwirken, die während ihres Anziehens die Blockierung des flexiblen Verbindungsteils (3) im Inneren der Hülse (11) nach dem Unterspannungsetzen des flexiblen Verbindungsteils ermöglicht.

3. Zusätzliche Befestigungsvorrichtung (1), flexibles Verbindungsteil (3) und Vorrichtung (50) für eine Wirbelsäule, wobei die zusätzliche Befestigungsvorrichtung (1) es ermöglicht, mindestens eine Doppelgewindepedikelschraube (2) der Vorrichtung (50) für eine Wirbelsäule mit dem entsprechenden Knochensegment vor der Installation eines Osteosynthesestabs der Vorrichtung (50) für die Wirbelsäule zu verbinden,
wobei die zusätzliche Befestigungsvorrichtung (1) einen Hauptkörper (10) umfasst, umfassend einerseits erste Befestigungsmittel (20), die direkt oder indirekt mit dem Körper der entsprechenden Pedikelschraube (2) zusammenwirken, und andererseits zweite Befestigungsmittel (30), die den Durchgang und die Blockierung des flexiblen Verbindungsteils (3) nach einem Unterspannungsetzen des Letzteren gewährleisten, um einen zusätzlichen Verankerungspunkt zu bilden,
wobei der Hauptkörper (10) aus einem offenen Ring (80) ausgebildet ist, der einerseits erste Befestigungen (20), die die Blockierung des Rings an dem Außenumfang eines halbkugelförmigen Kopfes (5) der Doppelgewindepedikelschraube (2) gewährleisten, und andererseits zweite Befestigungsmittel (30) vorweist, die in der seitlichen Verlängerung des Rings (80) angeordnet sind, um das flexible Verbindungsteil (3) in einer Ebene zu positionieren und blockieren, die zu der vertikalen Achse der Pedikelschraube versetzt ist, **dadurch gekennzeichnet, dass** die ersten Befestigungsmittel (20) einem offenen flexiblen Blatt (81) gebildet sind, dessen Innenseite mit einer Nut (82) versehen ist, die mit Nasen (83) zusammenwirkt, die an dem Außenumfang des halbkugelförmigen Kopfes (5) der Doppelgewindepedikelschraube (2) eingerichtet sind, wobei das offene flexible Blatt (81) an seinem ersten freien Ende eine Öffnung (84) aufweist, während das zweite freie und gegenüberliegende Ende fest mit den zweiten Befestigungselementen (30) verbunden ist, die aus einem Verbindungsstück (85) gebildet sind, das an seiner Basis einen horizontalen Schlitz (86) und in einer vertikalen Richtung eine Spannschraube (87) aufweist.

## Claims

1. Additional securing device (1), flexible link (3) and spinal device (50), wherein the additional securing device (1) allows at least one double-threaded pedicle screw (2) of the spinal device (50) to be connected to the corresponding bone segment prior to fitting an osteosynthesis rod of said spinal device (50),
wherein the additional securing device (1) comprises a main body (10) comprising first securing means (20) which cooperate directly or indirectly with the body of the corresponding pedicle screw (2) and comprising second securing means (30) which ensure the passage and immobilization of the flexible link (3) after it has been tensioned in order to constitute an additional anchoring point,
wherein the main body (10) is formed by a sleeve (11), the first securing means (20) of which consist of a central bore (21) that allows it to be positioned on a hemispherically profiled intermediate head (5) of the double-threaded pedicle screw (2), while the second securing means (30) are arranged as a lateral extension of said sleeve (11) in order to position and immobilize the flexible link (3) in a plane which is offset from the vertical axis of said pedicle screw (2),
**characterized in that** the second securing means (30) are formed, as the extension of the sleeve (11), by a connector (34) having, at its base, two horizontal slots (37; 38) which pass through said connector in two distinct axial directions which intersect at the center of said connector (34).

2. Additional securing device (1) according to claim 1, **characterized in that** the connector (34) has, above the horizontal slots (37, 38), a vertical threaded bore (39) intended to cooperate with a clamping screw (36) which, when tightened, immobilizes the flexible link (3) inside the sleeve (11) after said flexible link has been tensioned.

3. Additional securing device (1), flexible link (3) and spinal device (50), wherein the additional securing device (1) allows at least one double-threaded pedicle screw (2) of the spinal device (50) to be connected to the corresponding bone segment prior to fitting an osteosynthesis rod of said spinal device (50),
wherein the additional securing device (1) comprises a main body (10) comprising first securing means (20) which cooperate directly or indirectly with the body of the corresponding pedicle screw (2) and comprising second securing means (30) which ensure the passage and immobilization of the flexible link (3) after it has been tensioned in order to constitute an additional anchoring point,
wherein the main body (10) is formed by an open ring (80) having the first securing means (20) which ensure the immobilization of said ring on the external periphery of a hemispherical head (5) of the double-threaded pedicle screw (2) and having second securing means (30) arranged as the lateral extension of said ring (80) in order to position and immobilize the flexible link (3) in a plane which is offset from the vertical axis of said pedicle screw, **characterized in that** the first securing means (20) consist of an open flexible strip (81), the inner face of which is provided with a groove (82) which cooperates with lugs (83) arranged on the external periphery of the hemispherical head (5) of the double-threaded pedicle screw (2), said open flexible strip (81) has an opening (84) at its first free end, while the second, opposite free end is rigidly connected to the second securing means (30), which consist of a connector (85) having a horizontal slot (86) at its base and a clamping screw (87) in the vertical direction.
